(19) Europäisches Patentamt
European Patent Office
Office européen des brevets

(11) Publication number: **0 103 403**
**B1**

(12) **EUROPEAN PATENT SPECIFICATION**

(45) Date of publication of patent specification: **28.02.90**

(21) Application number: **83304636.0**

(22) Date of filing: **11.08.83**

(51) Int. Cl.[5]: **C 07 D 471/04,**
C 07 D 455/02, C 12 P 17/18,
A 61 K 31/40, A 61 K 31/435
// (C12P17/18,
C12R1:465),(C07D471/04,
221:00, 209:00)

(54) **Enzyme inhibitors.**

(30) Priority: **19.08.82 US 409763**
**19.08.82 US 409764**
**19.08.82 US 409765**

(43) Date of publication of application:
**21.03.84 Bulletin 84/12**

(45) Publication of the grant of the patent:
**28.02.90 Bulletin 90/09**

(84) Designated Contracting States:
**AT BE CH DE FR GB IT LI LU NL SE**

(56) References cited:
**US-A-4 316 905**
**US-A-4 316 906**

**NATURE, vol. 288, no. 5788, 20th November 1980, Macmillan Journals Ltd.; A.A. PATCHETT et al.: "A new class of angiotensin-converting enzyme inhibitors", pp. 280-283**

(73) Proprietor: **ELI LILLY AND COMPANY**
**307, East McCarty Street**
**Indianapolis Indiana 46285 (US)**

(72) Inventor: **Mynderse, Jon Stuart**
**4025 Rommel Drive**
**Indianapolis Indiana 46208 (US)**
Inventor: **O'Connor, Sean Christopher**
**5230 Wethersfield Road**
**Jamesville New York 13078 (US)**
Inventor: **Nakatsukasa, Walter Mitsuo**
**2118 Crossman Drive**
**Indianapolis Indiana 46227 (US)**

(74) Representative: **Hudson, Christopher Mark et al**
**European Patent Attorney Erl Wood Manor**
**Windlesham Surrey GU20 6PH (GB)**

(56) References cited:
**CHEMICAL ABSTRACTS, vol. 103, 1985, Columbus, OH (US); J.S.MYNDERSE et al.: "Isolation of A58365A and A58365B, angiotensin converting enzyme inhibitors produced by Streptomyceschromofascus", p. 336, no. 137602n**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 103 403 B1

**Description**

Each year hundreds of thousands of people die annually as a result of hypertension. Many different means of treating this problem exist but because of overlapping mechanisms of action, specific control of hypertension without affecting various other biological or biochemical functions is difficult to obtain. Consequently, undesirable side effects frequently occur. Researchers, therefore, are constantly searching to find drugs which act in a specific manner to achieve the desired result. *See generally*, W. O. Faye, *Principles of Medicinal Chemistry*, Lea & Febiger, Philadelphia, 1974, pp. 377—398.

One mechanism involved in hypertension is the renin-angiotensin system. The decapeptide angiotensin I, previously named hypertensin and angiotonin, is converted by ACE, angiotensin-converting enzyme, to the octapeptide angiotensin II. The converting enzyme, ACE, splits off the C-terminal histadyl leucyl residue of angiotensin I to form angiotensin II. Angiotensin II is a potent vasoconstrictor and acts directly on the adrenal gland to stimulate the release of aldosterone, M. Bodanszky, M. A. Ondetti, *Peptide Synthesis*, John Wiley, New York, 1966, pp. 215—223, and Pumpus, "Angiotensin", *Renal Hypertension*, I, Page, J. McCubbin, eds. (Yearbook Medical Publishers, Chicago, IL, 1968), pp. 62—68 Angiotensin I is formed by the action of the enzyme, renin, on the substrate, angiotensinogen. The role of the renin-angiotensin system in the etiology of hypertension has been much studied. See, for example, *J. Med. Chem.*, *24* (4), 355—361 (1981) and references cited therein.

Synthetic compounds which inhibit the action of ACE and which have hypotensive properties have been described previously; for example, the mercaptoacyl derivatives of substituted prolines disclosed in U.S. Patent No. 4,316,904, and U.S. Patent No. 4,316,906; the mercapto-substituted derivatives of certain amino acids disclosed in U.S. Patent No. 4,154,936; the hydroxycarbamoyl derivatives of pipecolic acid disclosed in U.S. Patent No. 4,154,937; and the mercaptoacyl derivatives of certain azetidine-2-carboxylic acids disclosed in U.S. Patent No. 4,046,889.

This invention provides angiotensin-converting enzyme inhibitors designated arbitrarily as A—58365 factors A, B, and C, which are useful hypotensive agents. These ACE inhibiting factors may be produced by culturing a new strain of *Streptomyces chromofuscus* NRRL 15098, under submerged aerobic fermentation conditions in an aqueous nutrient culture medium containing assimilable sources of carbon, nitrogen, and inorganic salts. The factors are isolated from the filtered fermentation broth and may be separated and purified by chromatographic techniques including reverse phase high performance liquid chromatography.

The A—58365 factors are potent ACE inhibitors useful in the control of hypertension and are potentially useful in the treatment of congestive heart failure.

Therefore, in accordance with the invention a compound of Formula (I)

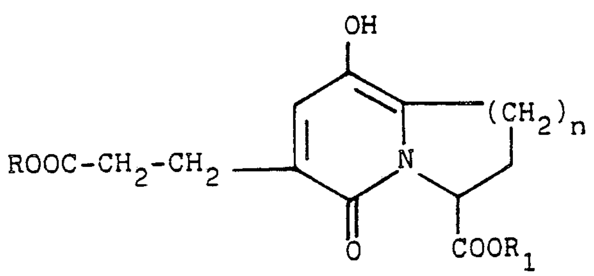

(I)

in which n is 1 or 2, R and $R_1$ may be the same or different, and are each hydrogen, $C_1$—$C_6$ alkyl, indan-5-yl, phthalidyl, or an acyloxymethyl group of the formula

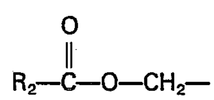

in which $R_2$ is $C_1$—$C_4$ alkyl, phenyl, halophenyl, or methylphenyl; or a pharmaceutically-acceptable salt thereof, is an angiotensin-converting enzyme inhibitor useful in the treatment of hypertension.

In a further aspect of the invention, there is provided a process for preparing A—58365 Factor A which comprises a process for producing A—58365 factors A, B, or C which comprises culturing *Streptomyces chromofuscus* NRRL 15098 in a nutrient culture medium containing assimilable sources of carbon, nitrogen and inorganic salts under submerged aerobic fermentation conditions, supplementing the culture medium with between about 1 to 6 g of proline per liter of medium.

In yet another aspect of the invention, there is provided a process for preparing A—58365 Factor B which comprises a process for producing A—58365 factors A, B, or C which comprises culturing *Streptomyces chromofuscus* NRRL 15098 in a nutrient culture medium containing assimilable sources of carbon, nitrogen and inorganic salts under submerged aerobic fermentation conditions, supplementing the culture medium with between about 1 to 6 g of proline and 1 to 3 g of lysine per liter of medium.

The ACE inhibiting compounds provided by this invention appear to be closely related chemically,

physically, and biologically. The following physical data and chemical properties characterize the individual factors obtained by the process of this invention.

A—58365 Factor A

Factor A is isolated, as described later, as a white amorphous powder and is highly soluble in water and polar organic solvents, such as methyl alcohol, ethyl alcohol, dimethylformamide and dimethylsulfoxide, but it is sparingly soluble in acetone and is insoluble in hydrocarbon solvents such as benzene and hexane.

Factor A absorbs radiation in the ultraviolet region of the spectrum. The following ultraviolet absorption spectrum of factor A was run on a Varian Cary spectrophotometer model 118. The spectrum was obtained on an aqueous solution of factor A under neutral, acidic, and alkaline conditions.

Neutral and acidic:

λmax 232 nm ε=6,000 (based on MW=267)
325 nm ε=7,600 (based on MW=267)

Alkaline:

λmax 243 nm ε=7,200
353 nm ε=7,400

Factor A has a characteristic fluorescence pattern. The following fluorescence spectrum of factor A was obtained with an Amino-Bowman Spectrophotofluorometer on an aqueous solution of factor A under acidic, netral, and alkaline pH.

Acidic:

Excitation maximum: 327 nm
Emission maximum: 398 nm

Neutral:

Excitation maximum: 324 nm
Emission maximum: 396 nm

Alkaline:

Excitation maximum: 350 nm
Emission maximum: 424 nm

The $^{13}$C NMR spectrum of factor A was obtained on a Bruker Model WM 270 NMR Spectrometer. The spectrum showed the following characteristic signals:

$^{13}$C NMR (67.9 MHz, $D_2O$): δ 25.66 (1C, t), 26.76 (1C, t), 27.66 (1C, t), 33.19 (1C, t), 63.80 (1C, d), 128.52 (1C, s), 134.72 (1C, d), 135.20 (1C, s), 136.06 (1C, s), 159.86 (1C, s), 174.42 (1C, s), 177.89 (1C, s).

The infrared absorption spectrum has the following significant absorption peaks:

| IR (KBr): | Frequency ($cm^{-1}$) | Intensity[1] |
|---|---|---|
| | 3500—2700 | br, s |
| | 2960 | w |
| | 2700—2400 | br, m |
| | 1719 | s |
| | 1660 | shd |
| | 1526 | s |
| | 1440 | m-w |
| | 1410 | m-s |
| | 1320 | s |
| | 1285 | m |
| | 1210 | m |
| | 1126 | w |
| | 1055 | vw |
| | 1028 | w |
| | 957 | vw |
| | 911 | w |
| | 878 | vw |
| | 836 | vw |

[1]/br=broad; m=medium; s=strong; w=weak; vw=very weak; m-s=medium to strong; m-w=medium to weak; shd=shoulder.

The characteristic signals of the $^1H$ NMR spectrum are shown below:
$^1H$ NMR (360 MHz, $D_2O$): δ 2.30 (1H, m), 2.57 (1H, m), 2.64 (2H, t), 2.74 (1H, dt), 2.81 (1H, dt), 3.05 (1H, dd), 3.15 (1H, ddd), 5.01 (1H, dd), 7.33 (1H, s).
The molecular weight of factor A is 267 as determined by the mass spectral analysis of factor A.
Fast atom bombardment (FAB): m/e 268
Field desorption: m/e 267, 223
High resolution FAB: m/e 268.081890; calculated 268.08211 for $C_{12}H_{14}NO_6$ $(M + H^+)$.
Elemental analysis carried out on a sample of factor A gave the following percent elemental composition based on the empirical formula of $C_{12}H_{13}NO_6$.
Calculated: C, 53.93; H, 4.90; N, 5.24
Found: C, 53.72; H, 5.10; N, 5.16.
Factor A has the following specific rotation: $[\alpha]_D^{25}$ (C=1%, $H_2O$) −199.5°
Electrometric titration carried out on a sample of factor A in 66% dimethylformamide showed the presence of three titratable groups: pK = 5.7, 7.5, 12.3.

A—58365 Factor B
The physical and spectroscopic properties of A—58365 factor B are presented in the following paragraphs. As indicated by the following data, factor B appears to be closely related to factor A, differing in structure by one methylene unit.
The infrared absorption spectrum of factor B contains the following significant absorption peaks:

| IR (KBr): | Frequency ($cm^{-1}$) | Intensity[1] |
|---|---|---|
| | 3600—2800 | br, s |
| | 2960 | m-w |
| | 2700—2400 | br, w |
| | 1717 | s |
| | 1652 | s |
| | 1538 | s |
| | 1436 | m-w |
| | 1412 | s |
| | 1350 | vw |
| | 1280 | m |
| | 1220 | m |
| | 1157 | w |
| | 1072 | w |
| | 1049 | w |
| | 1002 | w |
| | 906 | w |
| | 783 | m-w |
| | 639 | br, w |

[1]/Abbreviations are as noted hereinabove under factor A IR.

The proton magnetic resonance spectrum ($^1H$ NMR) for factor B contains the following signals:

$^1H$ NMR (360 MHz, $D_2O$): δ 1.67 (1H, m), 1.86 (1H, m), 2.08 (1H, m), 2.35 (1H, m), 2.66 (2H, t), 2.76 (1H, m), 2.78 (2H, m), 2.93 (1H, m), 5.10 (1H, dd), 7.35 (1H, s).

The carbon nuclear magnetic resonance spectrum of factor B has been run and the following signals are observed:

$^{13}C$ NMR (67.9 MHz, $D_2O$): δ 16.08 (1C, t), 23.43 (1C, t), 26.07 (1C, t), 26.36 (1C, t), 33.44 (1C, t), 58.03 (1C, d), 126.60 (1C, s), 132.88 (1C, s), 133.06 (1C, d), 137.14) (1C, s), 161.73 (1C, s), 176.97 (1C, s), 178.57 (1C, s).

Factor B absorbs radiation in the ultraviolet region of the spectrum and, like factor A, the maxima observed under neutral and acidic conditions are shifted to longer wave length upon the addition of base. The maxima obtained with an aqueous solution of factor B are as follows:

Neutral and acid: λmax 232 nm (ε=3,800), and λmax 333 nm (ε=5,600). The λmax at 232 nm shifts to 244 nm in base, while the λmax 333 nm shifts to 360 nm in base.

Factor B like factor A fluoresces blue under long wavelength ultraviolet radiation.

The molecular weight of factor B is 281 as determined by mass spectral analysis.

Field desorption mass spectrum: m/e 281 ($M^+$).

Based on analysis of the physical and spectral properties of factor B, the empirical formula is $C_{13}H_{15}NO_6$. Comparative analysis of the spectral data of factors A and B indicates that factor B is a higher homolog of factor A.

Factor B has solubility chracteristics similar to factor A. Factor B is highly soluble in water and polar organic solvents such as methyl alcohol, ethyl alcohol, dimethylformamide, and dimethylsulfoxide, but is sparingly soluble in acetone and is insoluble in hydrocarbon solvents such as benzene and hexane.

A58365 Factor C

Another factor produced in less abundance than factors A and B is obtained from the fermentation broth of *S. chromofuscus* NRRL 15098 and is designated as A—58365 factor C. Based on the characteristics

of this factor obtained thus far, factor C is similar to both factors A and B. Like the previously described factors, factor C fluoresces blue in long wave length ultraviolet radiation.

The molecular weight of factor C is 295 as determined by mass spectral analysis.

Field desorption mass spectrum: m/e 295 (M+).

Factor C has an empirical formula of $C_{14}H_{17}NO_6$.

The A—58365 factors, although similar, have characteristic retention times on high performance liquid chromatography (HPLC). The individual factors can be separated from one another via HPLC. THE HPLC system employed for the separation of the factors is a Waters Associates μ Bondapak C18, 4 mm × 300 mm column as the stationary phase. The mobile phase employed consists of 6% acetonitrile/0.3% formic acid/ 93.7% water at a flow rate of 2.5 ml/min. The characteristic fluorescence of the factors is used to detect their presence in the system. The fluoroescence is determined on a Schoeffel FS970 spectrophotometer.

The retention times of the factors in the above HPLC system are as follows:

| Factor | Retention (min.) |
|---|---|
| A | 4.88 |
| B | 12.47 |
| C | 18.21 |

The structures of the A—58365 factors A and B have been determined and are represented by the following structural formulas.

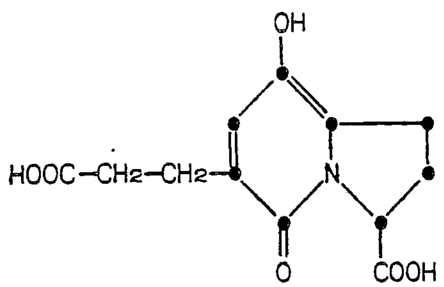

Factor A

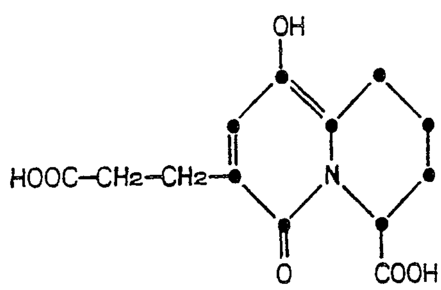

Factor B

This invention also provides for the A—58365 factors A and B in esterified form as well as in the salt form. Accordingly, this invention also provides a compound represented by the formula

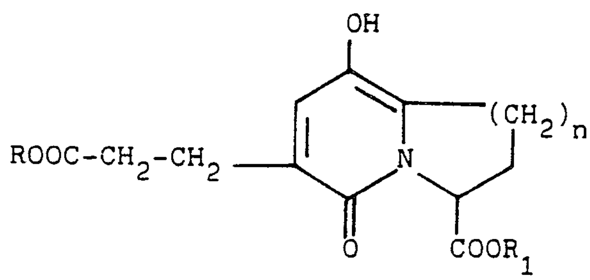

in which n is 1 or 2, R and $R_1$ can be the same or different and are each hydrogen, $C_1$—$C_6$ alkyl, indan-5-yl, phthalidyl, or an acyloxymethyl group represented by the formula

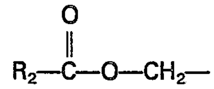

in which $R_2$ is $C_1$—$C_4$ alkyl, phenyl, halophenyl, or methylphenyl; or a pharmaceutically-acceptable salt thereof.

In the above formula the term "$C_1$—$C_6$ alkyl" refers to the straight and branched chain hydrocarbon radicals such as methyl, ethyl, n-propyl, iso-propyl, n-butyl, sec-butyl, t-butyl, n-amyl, iso-amyl n-hexyl, 1,1-dimethylbutyl, and like radicals. The term "$C_1$—$C_4$ alkyl- refers to methyl, ethyl, n-propyl, iso-propyl, n-butyl, sec-butyl, t-butyl, and like lower alkyl hydrocarbon radicals. The term "halophenyl" refers to the,

mono- or dihalophenyl groups such as 4-chlorophenyl, 3-chlorophenyl, 2-chlorophenyl, 3,4-dichlorophenyl, 3-bromophenyl, 4-bromophenyl, 2-fluorophenyl, 4-fluorophenyl, 3-iodophenyl, and the like; while the term "methylphenyl" refers to the mono and dimethylphenyl groups such as 4-methylphenyl, 3-methylphenyl, 2-methylphenyl, 2,6-dimethylphenyl, 2,4-dimethylphenyl, and like groups.

The A—58365 factors are esterified to form the respective mono and diesters by employing conventional esterification methods. For example, the compounds represented by the above formula in which R and $R_1$ are $C_1$—$C_6$ alkyl are prepared by the Fischer esterification method by reacting the diacid with a $C_1$—$C_6$ alcohol in the presence of an acid catalyst. Typical acid catalysts which can be used are boron trifluoride etherate, anhydrous hydrogen chloride, or p-toluenesulfonic acid. The esterification is carried out in an inert solvent which may be the $C_1$—$C_6$ alcohol itself or may be another solvent such as diethyl ether. In an example of the esterification, factor A is dissolved in methyl alcohol and about 1—3% anhydrous hydrogen chloride is bubbled into the solution. The acidic solution is then stirred from about 1—2 hours and the dimethyl ester is recovered.

Alternatively, the A—58365 factors can be esterified with the appropriate diazoalkane to obtain an ester of the invention.

The indan-5-yl esters represented by the above formula are prepared by esterifying the desired A—58365 factor with indane-5-ol for example by condensing the alcohol with the acid in the presence of a dehydrating agent such as a carbodiimide, for example, dicyclohexylcarbodiimide.

The phthalidyl esters of the A—58365 factors A and B are prepared by reacting an alkali metal salt of the factors with bromophthalide. The reaction can be carried out in a suitable solvent such as dimethylformamide or dimethylacetamide by reacting equimolar amounts of the salt and bromophthalide.

The esters of the above formula in which R or $R_1$ represents an acyloxymethyl group are prepared by reacting the A—58365 factor as the sodium or potassium salt with an acyloxymethyl halide represented by the formula

$$R_2\overset{\overset{\displaystyle O}{\|}}{C}—O—CH_2—X$$

in which X is preferably chloro or bromo and $R_2$ has the same meaning as defined above. Examples of acyloxymethyl chlorides and bromides which can be used are chloromethyl acetate, bromomethyl acetate, bromomethyl propionate, chloromethyl pivaloate, chloromethyl benzoate, bromomethyl 4-chlorobenzoate and like acyloxymethyl halides.

Following the preparation of diesters of factor A or factor B, any monoester present can be separated from the diester by solvent extraction at about pH 7. For example, a diester which contains a monoester is disolved in a water immiscible organic solvent such as methylene chloride and the solution is washed with a dilute solution of a base. The neutral to basic wash removes the monoester in the form of its water soluble salt.

The individual monoesters represented by the above formula can be obtained by esterifying the A—58365 factor with one equivalent of the alcohol. Any of the diester which is coproduced can be separated from the monoesters by solvent extraction at controlled pH as described above. The mixed monoesters obtained in the esterification are separated by conventional chromatographic methods for example, by preparative thin layer chromatography on silica gel or preferably, by HPLC.

The A—58365 factors are acidic compounds possessing two carboxylic acid functional groups. As with most carboxylic acids, the factors are capable of forming salts with suitable bases. Such salts include pharmaceutically-acceptable salts useful for the treatment of hypertension as well as salts which are useful in the isolation and purification of the individual factors. "Pharmaceutically-acceptable" salts are those salts useful in the chemotherapy of warm-blooded animals. Salts formed with the alkali metal and alkaline earth metal bases such as the sodium, potassium, and calcium salts can be formed by neutralizing an aqueous solution of the individual factors with the stoichiometric amount of the base followed by lyophilization of the aqueous salt soution. Suitable bases include, for example, the alkali metal carbonates, bicarbonates and hydroxides such as sodium carbonate, sodium bicarbonate, sodium hydroxide, potassium carbonate, potassium bicarbonate, potassium hydroxide, and calcium hydroxide. Salts of the factors can also be formed with suitable amines such as the primary and secondary amines such as methylamine, ethylamine, isopropylamine, n-butylamine, dimethylamine, cyclohexylamine, dicyclohexylamine, benzylamine, dibenzylamine, abietylamine, procaine, and like basic amines. The ammonium salt of the factors can also be obtained by conventional means. The amine salts can be obtained by adding a solution of the desired amine in a water miscible solvent to an aqueous solution of the factor. Many of the amine salts will precipitate from the aqueous solution or, if soluble in the mixture, can be obtained by lyophilization of the salt solution.

The A—58365 factors of this invention inhibit angiotensin I converting enzyme as demonstrated in *in vitro* studies using isolated guinea pig ileum. The *in vitro* tests were carried out as follows: segments (2—3 cm long) of the guinea pig ileum were mounted longitudinally in 10 ml. isolated tissue baths containing Krebs' solution having the following composition (mmol. concentrations): sodium chloride, 118.2; potassium chloride, 4.6; calcium chloride dihydrate, 2.5; monopotassium phosphate, 1.2; magnesium

sulfate, 1.2; dextrose, 10.0; and sodium bicarbonate, 24.8. In all experiments tissues were maintained at 37°C and were aerated with 95% oxygen and 5% carbon dioxide. The ilea were mounted between two electrodes consisting of a stainless steel rod (bottom) and a circular platinum wire (top). Square wave impulses (0.1 Hz) of supramaximal voltage (40 v) and 0.7 msec duration were provided by a Grass S44 stimulator. Tissues were equilibrated for approximately one hour at 1 g of applied force. Isometric responses were recorded on Beckman Dynographs.

Concentration-response curves to 3 or 4 concentrations of angiotensin I were generated. Tissues were then equilibrated with the A—58365 factor ($10^{-6}$ to $10^{-5}$ M) and concentration-response curves to angiotensin I were reassessed. Each tissue was given only one concentration of factor.

In all cases, a significant shift in the contractile response to angiotensin I occurred. The inhibition of ACE in guinea pig ileum was competitive. The data obtained with factors A and B in the above-described test also demonstrated that factors A and B did not inhibit acetylcholine release nor did they block cholinergic receptors. In the *in vitro* system, A—58365 factors A and B demonstrated pharmacological specificity to inhibit ACE.

The A—58365 factors of this invention are hypotensive agents which, by virtue of their ability to inhibit the enzymatic cleavage of angiotensin I to the pressor agent angiotensin II in mammalian tissue, are useful hypotensive agents. The ability of the ACE inhibiting factors of this invention to lower blood pressure was demonstrated with factor A in sodium depleted rats.

The compounds of this invention are useful for reducing blood pressure in a hypertensive mammal. The factors, or a pharmaceutically-acceptable salt thereof, may be administered individually or in any combination, e.g. factor A can be administered alone or in combination with factor B. In practicing the method, the A—58365 factor, or a pharmaceutically-acceptable salt thereof, is administered orally, intramuscularly, intravenously, or subcutaneously. For parenteral administration, the A—58365 factor, or a pharmaceutically-acceptable salt thereof, is dissolved in a physiologically-acceptable fluid for injection. Suitable physiological diluents such as Water For Injection, 0.9% saline, 5% glucose or other conventional diluent can be used. For oral administration, the A58365 factor, or a pharmaceutically-acceptable salt thereof, may be formulated as capsules, tablets or liquid suspensions. The A—58365 factor, or a pharmaceutically-acceptable salt thereof, can be administered in a non-toxic single daily dose of between about 100 mg./kg. and about 2,000 mg./kg. of body weight. Alternatively, it can be administered in multiple daily doses. The precise regimen will depend on such factors as the level of hypertension in the patient and the drug tolerance of the individual as well as other factors.

The A—58365 factor esters of this invention possess ACE inhibitory properties akin to the free acids. The acyloxymethyl esters as well as the indanyl and phthalidyl esters of the factors are potentially useful pro-drug forms of the factors useful in the preparation of formulations of the factors for oral use. Further, the $C_1$—$C_6$ alkyl diesters are useful in the isolation and purification of factors by chromatographic means.

The ACE inhibitory factors provided by this invention are prepared by culturing *Streptomyces chromofuscus* NRRL 15098 under aerobic fermentation conditions in an aqueous nutrient culture medium containing assimilable sources of carbon, inorganic salts and nitrogen. The culture medium employed in the fermentation may be any one of a number of media because the microorganism is capable of utilizing energy from a variety of nutrient sources. For example, a variety of carbohydrates including sugars and starches can be included in the culture medium to supply the carbon requirements of the microorganism. Likewise, various sources of nitrogen such as amino acids, distillers extracts, meat peptones, and casein hydrolysates can be employed in the culture medium. In the interest of economy in production, optimal yield, and ease of isolation of the ACE factors, certain culture media are preferred. For example, one of the preferred sources of carbon is potato dextrin, although various sugars such as glucose or fructose may also be used. Preferred sources of nitrogen are peptones and the hydrolysates of casein. Commonly, in the fermentation of microorganisms, nutrient inorganic salts can be incorporated in the culture medium for the production of the ACE factors. Such inorganic salts are the customary salts capable of yielding sodium, potassium, ammonium, clacium, phosphate, chloride, carbonate, and like ions. Trace elements may also be added to the fermentation medium; however, these are commonly available in sufficient trace amounts as constituents of other ingredients added to the media.

During the fermentation of *S. chromofuscus* NRRL 15098 to produce the A—58365 factors, cobaltous ion or other divalent cation is added to the fermentation medium. Cobaltous chloride is a convenient source of divalent cobalt. The divalent cation such as cobaltous ion is added in minor amounts; for example, the addition of between about 5 mg. and about 15 mg. of cobaltous chloride hexahydrate per liter of medium is sufficient.

It has been found that the production of the A—58365 factor A by *S. chromofuscus* is greatly enhanced by the addition of proline to the fermentation medium. Generally, between about 1 g. and about 6 g. of proline per liter of fermentation broth is sufficient. It has been found also that when, in addition to proline, the culture medium is supplemented with lysine, preferably L-lysine, A—58365 factor B is produced in enhanced yields and becomes the more abundant factor produced. Preferably in the production of factor B the culture medium is supplemented with between about 1 g. and about 6 g. of proline per liter and between about 1 g. and about 3 g. of lysine per liter of culture medium.

The A—58365 ACE inhibitory factor A is produced in surprisingly greater abundance by culturing *S. chromofuscus* NRRL 15098 under submerged aerobic fermentation conditions in an aqueous nutrient

culture medium supplemented with proline.

The amino acid proline used to supplement the production medium can be D-proline, L-proline, or D,L-proline. L-Proline is preferred because of its lower cost over either the D- or D, L forms of the amino acid. The proline used to supplement the medium also can be a component of a commercially available protein hydrolysate, enzymatic digest or other source of the amino acid.

In carrying out the process, between about 1 g and about 6 g of proline per liter of culture medium is employed. For best results in the production of the A—58365 factors and for reasons of economy, between about 3 g and about 4 g of proline per liter of culture medium are used.

The addition of proline or a source of proline to the *S. chromofuscus* NRRL 15098 culture medium results in at least a ten-fold increase in the amount of factor A produced in the fermentation. *S. chromofuscus* NRRL 15098 fermentations carried out without added proline produce about 1 mcg or less of A—58365 factor A per milliliter of filtered broth, while fermentations carried out in a culture medium supplemented with proline according to this invention produce about 10—12 mcg of factor A per milliliter of filtered fermentation broth.

In the proline supplemented culture, A—58365 factor A is the major factor i.e. produced in greater amount, and factors B and C are minor factors. It appears that the minor factors, A—58365 B and C may be produced in increased amounts in the improved process of this invention; however, they remain minor factors in the fermentation medium in comparison to the amount of factor A produced.

The fermentation can be carried out at temperatures between about 23°C and about 30°C. Best yields are obtained, however, when the fermentation is carried out at 25°C. During the fermentation the pH of the medium increases. Generally, the initial pH of the broth is adjusted to about 7, and the terminal pH is about 8 to 8.3.

To improve the yield of A—58365 factor B, one cultures the new strain of *Streptomyces chromofuscus* NRRL 15098 in an aqueous nutrient culture medium under submerged aerobic fermentation conditions, in which the culture medium is supplemented with between about 1 g and about 6 g of proline per liter and with between about 1 g and 3 g of lysine per liter. The nutrient culture medium employed contains the same sources of carbon, nitrogen and inorganic salts as previously described and, in addition, the amino acids proline and lysine.

In this process, fermentation can be carried out at a temperature between about 25°C and about 35°C. The fermentations carried out at about 30°C afford the highest assays for factor B and, accordingly, about 30°C is the preferred temperature.

In this aspect of the invention, the amino acids proline and lysine can be used in the L-form, the D-form or in the D,L-form. Other sources of the amino acids such as protein hydrolysates and enzymatic digests containing proline and lysine can be added to the culture medium to supply the amino acids. Because of their lower costs, L-proline and L-lysine are the preferred forms of the amino acids. Both of the amino acids, proline and lysine, are required constituents of the culture medium for the enhanced production of factor B. The fermentation carried out with only incorporated proline provides only surprisingly higher yields of A—58365 factor A while fermentations carried out only with a lysine supplemented culture medium do not have any appreciable effect on the yields of either factor.

Preferably, between about 3 g and about 4 g of proline and about 2 g of lysine per liter of culture medium are employed in this process.

Prior to the discovery of this process for improving the yield of Factor B, the proline supplemented *S. chromofuscus* NRRL 15098 culture medium provided on fermentation about 10—12 mcg/ml of factor A and about 2—3 mcg/ml of the minor factor B. A—58365 factor C occurs in even lesser amounts than factor B. When *S. chromofuscus* NRRL 15098 is cultured according to the lysine enriched process of this aspect of invention, assays show that about 5 mcg/ml of factor B are produced while between about <1 mcg/ml and about 2 mcg/ml of factor A are produced.

Accordingly, this invention provides a process for obtaining as the most abundant angiotensin converting enzyme inhibitor, A—58365 factor B.

Factor B is recovered from the filtered fermentation broth and separated from factor A and purified by following the isolation procedures described below.

In either case, the fermentation is carried out under aerobic conditions. Sterile air is passed through the fermentation medium with stirring during the course of the fermentation. For best results, the dissolved oxygen level in the fermentation medium should be maintained at approximately 30 to 40% of air sasturation.

An antifoam agent is generally useful to prevent excess foaming and any of the commonly employed antifoam agents such as the silicone antifoam agents can be employed in the fermentation.

The production of the ACE inhibitory factors during the course of the fermentation is followed by high performance liquid chromatography assay of an aliquot of the broth withdrawn from time to time. Peak production generally occurs between about 70 and about 90 hours into the fermentation. The assay is carried out employing as the stationary phase a 4 mm. × 300 mm. µ Bondapak C18 column (Waters Associates), and a mobile phase comprising acetonitrile:formic acid:water (6:0.3:93.7, v:v:v). The flow rate is 2.5 ml./min. Detection of the factors is carried out with a Schoeffel model FS970 spectrofluorometer by employing the wave length λexc = 327 nm and a 370 nm. emission cut off filter.

In carrying out the fermentation, a small volume of vegetative medium is inoculated with a lyophilized

pellet of *S. chromofuscus* NRRL 15098. Incubation of the culture is carried out at about 30°C. and, following the attainment of good growth which generally occurs in about two days, the vegetative medium or portions thereof are employed to inoculate a larger scale medium known as a 'bump" medium. The "bump" medium is an intermediate-size medium used as a large inoculum for large scale fermentation tanks. In general, the "bump" medium has the same or approximately the same composition as the vegetative medium. The initial small-volume vegetative medium can be a highly nutritive medium used for culturing microorganisms. A suitable vegative medium which provides good growth of *S. chromofuscus* NRRL 15098 is composed of trypticase soy broth plus approximately 1% glucose. Trypticase soy broth is a commercially available soybean-casein digest containing pancreatic digest of casein, soy peptone (a digest of soybeans), sodium chloride, dipotassium phosphate and glucose.

Alternatively, the lyophilized pellet of *S. chromofuscus* can be grown initially on an agar slant and, following growth, spores from the agar slant are transferred under sterile conditions to a vegetative medium. The growth vegetative medium can then be employed as described above for the inoculation of intermediate size "bump" media.

The microorganism employed in the method for producing the ACE inhibitors of this invention has been identified as a new strain of *Streptomyces chromofuscus* (Preobrashenskaya, Blinov and Ryabova 1957), Pridham, Hessetine and Benedict, "A guide for the Classification of Streptomycetes According to Selected Groups", *Appl. Microbiol. 6*: 52—59, (1957).

The new strain of *S. chromofuscus* has been deposited (June 23, 1982) without restriction as to public availability, in the permanent collection of the Agriculutural Research Culture Collection, Northern Regional Research Center, Department of Agriculture, 1815 North University Street, Peoria, IL 61604, where it has been assigned the accession number NRRL 15098.

The parent culture from which the new strain was selected was isolated from a soil sample collected in Brazil, South America.

The following paragraphs and tables contain the taxonomic description of the new strain as determined by standard methods and by comparisons with published taxonomic descriptions of *Streptomyces* resembling the new strain.

The methods recommended by the International *Streptomyces* Project (ISP) for the characterization of *Streptomyces* species have been followed along with certain supplementary tests, Shirling, E. B. and Gottlieb, D., 1966, "Methods of Characterization of *Streptomyces* Species," *Int. J. Syst. Bacteriol. 16* (3), pp. 313—340.

Cultural Characteristics of *S. chromofuscus* NRRL 15098

Table 1 below contains the description of the growth of the new strain on various culture media. The color names in the Table were assigned by reference to the ISCC—NBS Centroid Color Charts, standard sample No. 2106, U.S. Department of Commerce, National Bureau of Standards, 1958; and Tresner, H. D. and Backus, E. J., 1956, "System of Color Wheels for Streptomycete Taxonomy", *Appl. Microbiol., 11*, 335—338; and to the *Color Harmony Manual*, 4th edition, Color Standards Department, Container Corporation of America, Illinois, 1958. In summary, *S. chromofuscus* NRRL 15098 produces an abundant serial mycelia having a spore mass color in the gray (GY) color series. The nearest matching color tab for the gray color series in the Tresner and Backus system is *2 dc* yellowish gray to *2 fe* medium gray. In the ISCC—NBS system, the nearest matching color chip is 112, light olive gray and 93, yellowish gray. This cultural feature is produced on yeast-malt extract agar (ISP No. 2), oatmeal agar (ISP No. 3), glycerolasparagine agar (ISP No. 5), Czapek's solution agar and tomato paste oatmeal agar. This growth and color feature is observed best when grown on inorganic salts-starch agar (ISP No. 4). The color of the reverse side is yellow-brown. This reverse color is unaffected by pH. No soluble pigment is produced in any media except ISP No. 5, where a light yellow pigment is present.

TABLE 1

Cultural Characteristics of *S. chromofuscus NRRL 15098*

| Medium | Growth Characteristic[1] |
|---|---|
| ISP No. 2[2] | Abundant growth; abundant aerial mycelia: 2dc Yellowish Gray (GY); reverse: 69; deep OY; no soluble pigment. |
| ISP No. 3 | Fair growth; reverse: 91.d.gy.Y; poor aerial mycelial growth: *5fe* Light Grayish Reddish; no soluble pigment. |
| ISP No. 4 | Abundant growth; reverse: 5b. deep Br; abundant aerial mycelia: *2fe* Medium Gray (GY); no soluble pigment. |
| ISP No. 5 | Fair growth; reverse: 94.1.01 Br; Fair aerial mycelial development: *2dc* Yellowish Gray (GY); Light Yellow soluble pigment. |
| Czapek's Agar | Fair growth; reverse: 79.1.gy.yBr; Fair aerial mycelial development: *3ge* Light Grayish Yellowish Brown (GY); no soluble pigment. |
| TPO[3] | Abundant growth; reverse: 75. deep yBr; abundant aerial mycelia: *3ge* Light Grayish Yellow Brown (GY); no soluble pigment. |

[1]/The numbers and letters used for reverse side colors refer to the color charts of ISCC—NBS Centroid Color Charts Standard Sample No. 2106, U.S. Department of Commerce, National Bureau of Standards. The underlined numbers and letters used to designate color of the aerial mycelia refer to Tresner, H. D. and Backus, E. J. 1956, "System of Color Wheels for Streptomycete Taxonomy", *Appl. Microbiol. 11*: 335—338. (GY) refers to the Gray series.
[2]/ISP = International Streptomyces Project Media
[3]/TPO = Tomato paste oatmeal agar

Morphological Characteristics
of *S. chromofuscus* NRRL 15098

The new strain of *S. chormofuscus* produces well-developed, non-fragmented mycelia which are monopodially branched. Sporophores are produced on aerial hyphae forming spirals of 4—5 coils. Some spirals form very tight balls at the end of the sporophore. These balls could be mistaken for sclerotio. Spores are oblong with a spiny surface. The new strain is placed in the Spirales (S) section of Pridham, et al., Pridham, T. G., Hesseltine, C. W., and Benedict, R. G., 1957, "A Guide for the Classification of Streptomycetes According to Selected Groups", *Appl. Microbiol. 6*: 52—79.

Morphology was studied with an optical light microscope. A scanning electron microscope (SEM) was used to study the spore surface and ornamentation.

Physiological Characteristics
of *S. chromofuscus* NRRL 15098

Analysis of hydrolyzed whole cells of the *S. chromofuscus* strain indicated the presence of LL-diaminopimelic acid. No *meso* isomer was detected. Sugar analysis of hydrolyzed whole cells showed the presence of glucose, mannose, ribose, and rhamnose. These results show that the cell wall of the strain is a Type I cell wall with no characteristic sugar pattern and is indicative of the genus *Streptomyces*, Buchanan, R. E. and Gibbons, N. E. (eds.) 1974, *Bergey's Manual of Determinative Bacteriology*, 8th edition, The Williams and Wilkins Co., Baltimore, MD.

The carbon utilization pattern for *S. chromofuscus* NRRL 15098 is shown in the following Table 2. Carbon utilization was determined with ISP basal medium to which sterilized carbon sources were added to equal a final concentration of 1.0 percent. Plates were incubated at 30°C. and were read after 14 days.

TABLE 2

Carbon Utilization of *S. chromofuscus* NRRL 15098

| Carbon Source | Utilization[1] |
|---|---|
| No carbon | − |
| L-arabinose | + |
| D-fructose | + |
| D-glucose | + |
| i-inositol | + |
| D-mannitol | + |
| raffinose | − |
| L-rhamnose | + |
| sucrose | ± |
| D-xylose | + |
| D-arabinose | + |
| cellobiose | + |
| D-galactose | + |
| lactose | + |
| D-maltose | + |
| melbiose | + |
| sodium acetate | − |
| sodium citrate | + |
| sodim succinate | + |
| D-ribose | + |
| salicin | + |

[1]/ − = no utilization
+ = utilization
± = doubtful utilization

*S. chromofuscus* NRRL 15098 will hydrolyze starch and partially hydrolyze skim milk. A dark brown growth ring is formed on skim milk. Gelatin is not hydrolyzed by the new strain. In ISP No. 8, organic nitrate broth, the strain fails to reduce nitrates to nitrites.

The new strain will tolerate up to about 7 percent sodium chloride.

The new strain will grow at temperatures between about 15°C. and about 40°C.

TABLE 3
Melanoid Pigment Formation
of *S. chromofuscus* NRRL 15098

| Medium | Pigment Formation |
|---|---|
| ISP No. 1 tryptone-yeast extract broth | + |
| ISP No. 6 peptone-yeast extract iron agar slant | + |
| ISP No. 7 tyrosine agar | + |
| ISP No. 7 agar slants without tyrosine | − |

Based upon comparisons of the morphological, cultural, and physiological characteristics of the A—58365-producing organism with the published descriptions of similar species, the culture described herein was most similar to *S. chromofuscus*, Shirling, E. B. and Gottlieb, D., 1968, "Cooperative Description of Type Cultures of Streptomyces", *Int. J. Syst. Bacteriol. 18* (4): 279—392. However, the A—58365 organism differs from the published species and is therefore classified as a new strain thereof. Although having many similar characteristics, the known *S. chromofuscus* strain differs from the A—58365 strain provided herein by not utilizing the sugar raffinose and by the lack of melanoid pigment production on ISP No. 7 agar.

*S. chromofuscus* NRRL 15098, in addition to producing the ACE inhibitors described here, also produces in minor amounts the antibiotic asukamycin. AM1042 and another antibiotic metabolite which may be methylenomycin. Asukamycin is described by Omura, U.S. Pat. No. 4,226,879, and is produced by *Streptomyces nodosus* NRRL 8185. *S. nodosus* differs culturally from *S. chromofuscus* NRRL 15098 in its distinctive reverse and soluble pigmentation. Morphological differences are: poor sporophore development, short spore chain, and smooth spore surface ornamentation. Physiologically *S. nodosus* differs from the new strain herein in not producing melanoid pigments in ISP No. 1 broth or on ISP No. 6 and ISP No. 7 agar slants. These differences show that *S. nodosus* is a separate and distinct species from the organism described here.

The other antibiotic metabolite produced during the growth of *S. chromofuscus* NRRL 15098 has been tentatively identified as methylenomycin. This antibiotic, previously reported to be produced by *Streptomyces violaceoruber* 2416, is described by Haneishi, T., et al., *J. Antibiotics, 27*, 386—392 (1974).

In a further aspect of this invention there is provided a biologically pure culture of *S. chromofuscus* NRRL 15098 which in addition to the foregoing cultural, morphological and physiological characteristics also produces the ACE inhibitors described here.

The A—58365 factors A and B are isolated from the fermentation medium and are separated from one another by chromatography. The isolation and separation of the factors is carried out as follows. The whole fermentation broth is acidified to about pH 2.0 and is filtered to remove the mycelium and other insolubles. Desirably, a filter aid is used to enhance the rate of filtration. Following the filtration, the pH of the filtered broth is adjusted to about pH 7.0 with a base such as sodium hydroxide. To remove inactive neutral impurities, the neutral filtered broth is treated with a nonfunctional polymeric reticular resin, for example, a polystyrene resin such as Diaion HP—20 (Mitsubishi Chemical Co.) or a XAD resin (Rohm and Haas, Philadelphia PA). The neutral broth may be passed through a column packed with the resin or, alternatively, the resin may be added to the neutral broth in a suitable vessel and stirred with the broth to adsorb inactive impurities. In this preliminary purification, the amount of resin used corresponds to about one-tenth of the volume of the broth. Preferably, the resin is stirred in the neutral broth for about two hours and is then separated by filtration. The pH of the resin-treated broth is then lowered to about pH 2.0—3.0 with an acid such as hydrochloric acid and the acidified broth is chilled and filtered to remove any inactive precipitate.

Then, the acidic, polished broth is chromatographed over a nonfunctional resin, preferably Diaion HP—20. The acidic broth is poured onto a column packed with the HP—20 resin and the effluent discarded. After the column is washed with a dilute acid, preferably 0.3% aqueous formic acid, the A—58365 factors are eluted by gradient elution employing a gradient from water:formic acid (99.7:0.3, % by vol.) to acetonitrile: water:formic acid (20:79.7:0.3, % by vol.). Multiple fractions are collected with factor A eluting in the early active fractions and factors B and C eluting in the later active fractions. The course of the chromatography is followed by the HPLC assay described earlier.

The A—58365 factor A containing fractions are pooled and concentrated by evaporation. The concentrate is then applied to an acidic resin such as a polystyrene sulfonic acid resin in the acid cycle, for example, Dowex 50W resin (Dow Chemical Co.). Factor A is washed from the resin with deionized water in multiple fractions. The factor A containing fractions are combined and concentrated by evaporation.

The acidic (pH 2—3) concentrate of factor A is filtered and then subjected to reverse phase preparative HPLC on $C_{18}$ silica gel such as octadecylsilanized Whatman LP—1 or Water's Assoc. $C_{18}$ silica gel. The column is developed first with aqueous formic acid (0.3:99.7% by vol.), next with acetonitrile:formic acid:water (1.0:0.3:98.7% by vol.), and finally with acetonitrile:formic acid:water (2.5:0.3;97.2% by vol.). The fractions containing factor A are pooled and concentrated by evaporation.

The factor A concentrate from the HPLC column is chromatographed over an anion exchange resin such as 100—200 mesh BioRex 5 resin in the chloride cycle (BioRad Laboratories, Richmond, CA). The column is eluted with about 0.2M to about 0.5M sodium chloride and pure factor A containing fractions are combined. The pooled fractions are acidified to about pH 2.2—2.5 with acid, eg. 1N hydrochloric acid, and are passed over Diaion HP—20 resin to remove salts present in the fractions from the anion exchange chromatography. Prior to use, the HP—20 resin is prepared wth 0.01N hydrochloric acid. Factor A is eluted by first washing the column with dilute acid (pH 2.3), next with water and finally with aqueous acetonitrile (15:85% by volume). The factor A containing fractions are pooled, concentrated by evaporation, and the concentrate is lyophilized to provide pure factor A as an amorphous white powder.

The combined fractions containing factors B and C, obtained as described above by gradient elution from the HP—20 chromatography of polished broth, are concentrated to a smaller volume by evaporation. The concentrate of factors B or C is next chromatographed over a polystyrene polysulfonic acid resin such as Dowex 50W ($H^+$) (Dow Chemical Co.). Factors B or C are washed from the resin with deionized water. The fractions containing factor B are combined and concentrated. Factor C containing fractions are likewise combined and concentrated.

The concentrates of factors B and C are separately purified by reversed phase HPLC using $C_{18}$ silica gel eg., octadecylsilanized Whatman LP—1 silica gel. Factor B is eluted using first formic acid:water (0.3:99.7% by vol.), next acetonitrile:formic acid:water (6.0:0.3:93.7%), and finally acetonitrile: formic acid:water (15.0:0.3:84.7% by vol.). Factor C may be eluted using similar solvent systems containing, however, a higher concentration of acetonitrile in the solvent mixture. The active fractions are combined and concentrated.

The concentrates of factors B and C can be further purified by following the procedures described above for factor A. For example each concentrate is chromatographed over an anion exchange resin, the respective eluents acidified, desalted over a non-functional resin, and the desalted eluents lyophilized.

Factor A is produced in greater abundance than factors B and C and is the preferred ACE inhibitor of this invention. Factor B in turn is produced in greater amounts than is Factor C.

As mentioned, the antibiotic asukamycin and another antibiotic metabolite (possibly methylenomycin) are produced in minor amounts along with the ACE inhibitory factors. During the isolation and purification of the A—58365 factors described above, the antibiotic metabolites are lost.

The following non-limiting Examples are provided to illustrate further the invention.

## Example 1

Production and Isolation of A—58365 Factors

A lyophilized pellet of *S. chromofuscus* NRRL 15098 was used to inoculate 50 ml. of a sterilized vegetative medium of three percent trypticase soy broth containing one percent glucose. The inoculated medium was incubated at 30°C. for 48 hours with shaking. This vegetative medium was used to inoculate a bump media as follows: Two, 2,000 ml. flasks each containing 400 ml. of three percent trypticase soy broth with one percent glucose added were each inoculated with 10 ml. of the vegetative medium. The bump cultures then were incubated for 24 hours at a temperature of 30°C.

Both of the bump cultures were used to inoculate 100 liters of a production medium having the following composition:

| Ingredient | | Concentration (g./l.) |
|---|---|---|
| Dow-Corning antifoam A | | 0.2 |
| Potato Dextrin | | 35 |
| Yeast | | 0.25 |
| OM Peptone[1] | | 20 |
| $COCl_2 \cdot 6H_2O$ | | 0.01 |
| L-Proline | | 4 |
| N—Z Amine A[2] | | 4 |
| Deionized water | qs. to | 100.1. |

[1]/OM Peptone is a soluble meat peptone, Amber Laboratories, Juneau, WI
[2]/N—Z Amine A is an enzymatic hydrolysate of casein, Humko Sheffield Chemical, Lyndhurst, NJ.

The pH of the medium was adjusted to 7.0 with 5N sodium hyroxide before sterilization. After sterilization the medium was inoculated with the bump media described above and the production fermentation was allowed to proceed at a temperature of 25°C. for 90 hours. During the fermentation sterile air was passed through the medium, with stirring, at a rate sufficient to maintain the dissolved oxygen content of the medium at about 30% to about 40% of air saturation.

The pH of the medium increased during the fermentation to a final pH of 8.3.

During the fermentation the medium was assayed for A—58365 factor content by using the HPLC system described earlier. The factors were detected by fluoroscence using a Schoeffel Model FS970 spectrophotofluorometer at the wave length λexc.= 327 nm with a 370 nm cutoff filter. The production medium assayed for about 11.5 mcg./ml. after the 90 hour fermentation period.

Three, 100-liter fermentations carried out as described above were acidified separately in the fermentors, to pH 2.0 with concentrated hydrochloric acid. The acidified whole broths were combined and filtered with the aid of 2% Hyflo filter aid. The pH of the filtered broth was adjusted to 7.0 with 5N sodium hydroxide. The nonfunctional resin Diaion HP—20 was added to the neutral broth in an amount corresponding to one-tenth the volume of the filtered broth and the resin-broth mixture was stirred for two hours. The broth was separated from the resin and acidified to pH 2.0 with 5N hydrochloric acid. The acidified broth was chilled and filtered to remove inactive precipitates. The acidified broth was applied to a 20′ × 4″ i.d. column containing 20 l. of Diaion HP20 and the effluent discarded. The column was next washed with 60 l. of 0.3% aqueous formic acid and the effluent discarded. The A—58365 factors were then eluted with a 100 l. gradient from water-formic acid (99.7:0.3; v:v:v:) to acetonitrile-water-formic acid (20:79.7:0.3; v:v:v.) and 2 l. fractions were collected. Fractions 27 to 48 containing factor A were pooled and concentrated *in vacuo* to a volume of 750 ml. Elution was continued with 20 liters of acetonitrile-water-formic acid (20:79.7:0.3). Fractions 56 to 63 containing factor B were pooled and concentrated to a volume of 350 ml.

The factor A containing concentrate was applied to a 9.3 cm × 80 cm (5 l.) column of Dowex 50W × 2 ($H^+$) and the column was eluted with about 17 l. of deionized water. One-liter fractions from 9 to 15 liters of eluted volume containing factor A were collected, pooled and concentrated to about 200 ml.

The factor A concentrate (pH 2—3) was filtered and chromatographed on reverse phase HPLC on a 8 cm. × 1 m. column (Jobin Yvon Chromatospac Prep instrument) containing aprpoximately 2.5 kg. (4—4.5 l.) of octadecylsilanized Whatman LP—1 silica gel. The column was developed first with two liters of formic acid-water (0.3:99.7, v:v), then with 5 liters of acetonitrile-formic acid-water (1.0:0.3:98.7, v:v:v), and, finally with 20 l. of acetonitrile-formic acid-water (2.5:0.3:97.2, v:v:v). Fractions of 500 ml. volume were collected. Fractions 32—44 containing factor A were pooled and concentrated by evaporation to a volume of 200 ml.

The factor A concentrate from HPLC was applied to 2.5 cm × 30 cm. (180 ml.) column of 100—200 mesh BioRex 5 ($Cl^-$) resin (BioRad Laboratories, Richmond, CA). The resin was washed with deionized water and both the wash and effluent were discarded. The column was developed with 400 ml. of 0.20M sodium chloride and then with 2200 ml. of 0.35M sodium chloride. Fractions of 20 ml. in volume were collected and fractions 106—140 containing pure factor A were pooled. The pH of the pooled fractions was adjusted to 2.3 with 1N hydrochloric acid and the acidified pool was applied to a 2.8 cm. × 19 cm. (120 ml.) column of Diaion HP20 set in 0.01N hydrochloric acid. The column was washed first with 100 ml. of deionized water acidified to pH 2.3 with dilute hdyrochloric acid, then with 220 ml. of deionized water pH 5.9), and was then eluted with 340 ml. of acetonitrile-water (15:85, v:v). After multiple fractions totaling about 180 ml. in volume has been collected, the fractions from 0—180 ml. of effluate were collected,

combined, concentrated by evaporation and lyophilized to give 1.31 g. of pure factor A.

The concentrate of pooled factor B containing fractions, eluted from the Diaion HP—20 column as described above, was applied to a 9.3 cm × 80 cm. (5 l.) column of Dowex 50W × 2 ($H^+$ cycle) resin. The column was eluted with 32 l. of deionized water and factor B was collected in one liter fractions from 10 to 14 liters to eluate. The active fractions were combined and concentrated to a volume of about 250 ml.

The A—58365 factor B containing concentrate was subjected to the same reverse phase HPLC as described above for the purification of factor A. The column was developed first with two liters of formic acid-water (0.3:99.7% by vol.), next with acetonitrile-formic acid-water (6.0:0.3:93.7% by vol.) and then with acetonitrile-formic acid-water (15.0:0.3:84.7, % by vol.). Multiple fractions of about 500 ml. were collected. Fraction 24 containing factor B was concentrated by evaporation to a volume of 100 ml.

The concentrate of factor B was further purified on an anion exchange resin as follows. A 2.0 cm i.d. × 25 cm column packed with BioRex 5 ($Cl^-$) anion exchange resin was charged with the concentrate and the column was eluted first with 200 ml of 0.2M sodium chloride followed by elution with 1600 ml of 0.35M sodium chloride. Multiple fractions of 10 ml volume were collected and fractions 193—210 were pooled. The pH of the pooled fractions was adjusted to pH 2.3 with 1N hydrochloric acid and the acidified pool was applied to an 8 mm i.d. × 20 cm (10 ml). Diaion HP—20 column set in 0.01N hydrochloric acid. The column was washed first with 300 ml of deionized water (adjusted to pH 2.3) then with 14 ml of deionized water (pH 5.9). The effluent and wash were discarded and factor B was eluted with 44 ml of acetonitrile-water, (15:85% by vol). Multiple fractions of 2 ml volume were collected. Fractions 8 to 11 were pooled and concentrated to a volume of 1 ml. The concentrate was lyophilized to give 2.9 mg of pure factor B.

## Example 2

Production of A—58365 Factors A and B With Proline and Lysine Supplemented Culture Medium

*S. chromofuscus* NRRL 15098 was cultured in vegetative medium prpeared and cultured as described by Example 1 and the culture medium was used to inoculate a production medium having the following composition.

| Ingredient | Concentration (g/l) |
|---|---|
| Potato Dextrin | 25 |
| NZ Amine A[1] | 3 |
| Yeast | 0.25 |
| L-Proline | 3 |
| Beef Extract | 1 |
| CO $Cl_2 \cdot 6H_2O$ | .01 |
| L-Lysine | 2 |
| Deionized water | qs 1000 ml |

[1]Enzymatic hydrolysate of casein, Humko Sheffield Chemical, Lyndhurst, NJ.

The pH of the medium was adjusted to 7.0 with 5N sodium hydroxide prior to sterilization. After sterilization the medium was inoculated and cultured at a temperature of 30°C for 96 hours.

The fermentation medium was assayed for factor content during the fermentation by employing the HPLC system described earlier.

Factor B is recovered from the filtered fermentation broth, isolated, and purified by the chromatographic procedures described in Example 1.

## Example 3

A—58365 Factor A dimethyl ester

*Method A*

To a solution of 319 mg of A—58365 factor A in 7.5 ml of methyl alcohol were added 2.5 ml of methyl alcohol containing 4 percent by weight of hydrogen chloride and the solution was stirred at room temperature for 140 minutes. The esterification mixture was then evaporated to dryness and the residue dissolved in approximately 20 to 25 ml of water. The pH of the solution was adjusted to 6.8 and left standing at room temperature overnight. After standing the pH of the aqueous solution was adjusted from 6.2 to 7.1 and the solution was then extracted three times with 100 ml portions of methylene chloride. The combined extracts yielded about 151 mg of factor A dimethyl ester.

*Method B*

A solution of 35 mg of factor A in 4.5 ml of a solution of boron trifluoride in methyl alcohol (14% $BF_3$ in anhydrous methyl alcohol, available from Pierce Chemical Co., Rockville, IL) was heated with stirring at a temperature of about 65°C for 2.5 hours. During the reaction, air was excluded from the reaction vessel. Thereafter, the reaction mixture was concentrated by evaporation to a volume of about 1.5 ml and the concentrate dissolved in 10 ml of water. The aqueous solution was extracted five times with equal volumes of methylene chloride, the extracts were pooled, washed with 5 ml of water, dried over sodium sulfate, filtered, and evaporated to dryness to yield 24.4 mg of factor A dimethyl ester.

Example 4

A—58365 Factor A diethyl ester, mono ethyl esters

A solution of 507 mg of factor A in 15 ml of absolute ethyl alcohol was cooled in an ice bath and 10 ml of absolute ethyl alcohol containing three percent by weight of hydrogen chloride was added with stirring. The reaction flask was flushed with nitrogen, stoppered, and allowed to stand at room temperature. The course of the esterification was followed by HPLC of small aliquots of the reaction mixture withdrawn from time to time. After 8 hours the reaction mixture was diluted with 50 ml of water and the pH of the aqueous solution adjusted to 6.9 with dilute sodium hydroxide. The solution was evaporated to remove the ethyl alcohol and the aqueous concentrate was diluted with water to a volume of 40 ml and treated with dilute sodium hydroxide to adjust the pH to 7.09. The solution was then extracted four times with 50 ml portions of methylene chloride and the extracts combined, washed with 20 ml of water, dried over sodium sulfate and evaporated to dryness *in vacuo* to yield 222 mg of the diethyl ester of factor A.

The pH of the aqueous phase retained from the above extraction of the diester was adjsuted to 1.95 with hydrochloric acid and extracted five times with 50 ml portions of methylene chloride. The extracts were combined, washed with dilute acid, and set aside. The acidic aqueous phase was extracted four times with 50 ml portions of ethyl acetate, and the extracts were combined and washed with dilute acid. The ethyl acetate extract and the methylene chloride extract were combined, dried over sodium sulfate and evaporated to dryness. There were obtained 257 mg of a mixture of the monoethyl esters of factor A. Analytical HPLC and nmr (360 MHz) showed a 9:1 mixture of the monoethyl esters.

**Claims for the Contracting States: BE CH DE FR GB IT LI LU NL SE**

1. A compound of Formula (I)

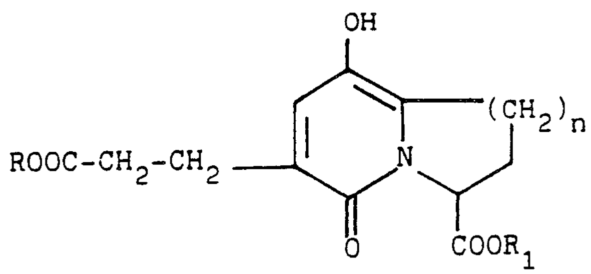

(I)

in which n is 1 or 2, R and $R_1$ may be the same or different, and are each hydrogen, $C_1$—$C_6$ alkyl, indan-5-yl, phthalidyl, or an acyloxymethyl group of the formula

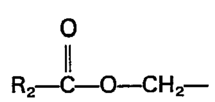

in which $R_2$ is $C_1$—$C_4$ alkyl, phenyl, halophenyl, or methylphenyl; or a pharmaceutically-acceptable salt thereof.

2. A compound of Formula (II) in which R and $R_1$ are defined as in Claim 1;

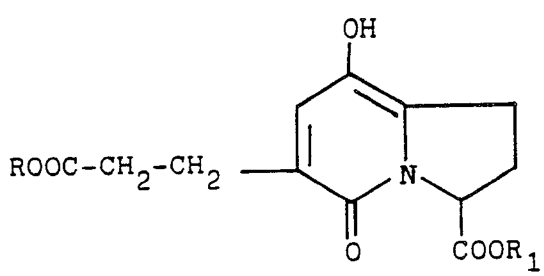

(II)

or a pharmaceutically-acceptable salt thereof.

3. A—58365 Factor A of Formula (III):

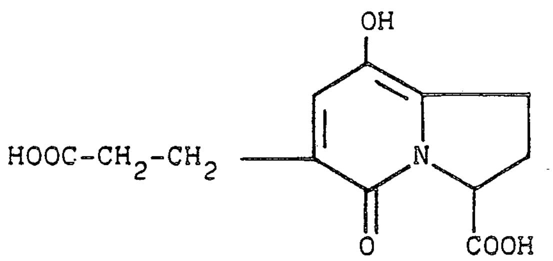

(III)

or a pharmaceutically-acceptable salt thereof.

4. A compound of Formula (IV) in which R and $R_1$ are defined as in claim 1;

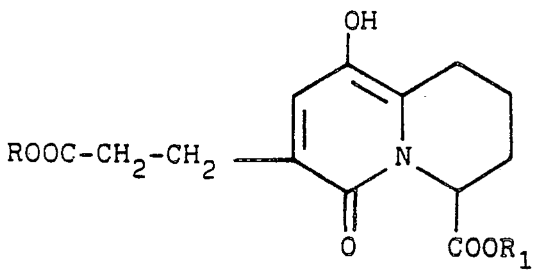

(IV)

or a pharmaceutically-acceptable salt thereof.

5. A—58365 Factor B of Formula (V):

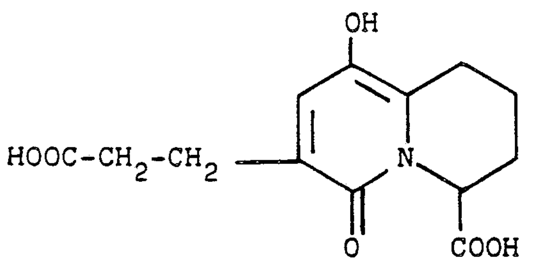

(V)

or a pharmaceutically-acceptable salt thereof.

6. A—58365 Factor C which

(a) has a molecular weight of 295;

(b) shows m/e 295 [$M^+$] in its field desorption mass spectrum;

(c) has an empirical formula of $C_{14}H_{17}NO_6$;

(d) a retention time of about 18.2 minutes upon reverse phase high performance liquid chromatography using a mobile phase of 6% acetonitrile, .3% formic acid 93.7% water;

or a pharmaceutically-acceptable salt thereof.

7. A process for producing A—58365 factors A, B, or C which comprises culturing *Streptomyces chromofuscus* NRRL 15098 in a nutrient culture medium containing assimilable sources of carbon, nitrogen and inorganic salts under submerged aerobic fermentation conditions.

8. A process for producing A—58365 Factor A as claimed in claim 7 which comprises supplementing the culture with between about 1 to 6 g of proline per liter of medium.

9. A process for preparing A—58365 Factor B as claimed in claim 7 which comprises supplementing the culture medium with between about 1 to 6 g of proline and 1 to 3 g of lysine per liter of medium.

10. A process as claimed in any one of claims 7 to 9 followed by isolating Factor A, Factor B or Factor C, and, optionally if desired, esterifying and/or salifying the product.

11. A compound of Formula (I) as defined in any of claims 1 to 6, or a pharmaceutically-acceptable salt thereof, for use as a hypotensive agent.

12. A pharmaceutical formulation which comprises as an active ingredient, a compound of Formula (I) as claimed in any of claims 1 to 6; or a pharmaceutically-acceptable salt thereof, associated with one or more pharmaceutically-acceptable carriers or vehicles therefor.

13. A biologically pure culture of *Streptomyces chromofuscus* NRRL 15098.

14. *Streptomyces chromofuscus* NRRL 15098.

**Claims for the Contracting State: AT**

1. A process for preparing a compound of Formula (I)

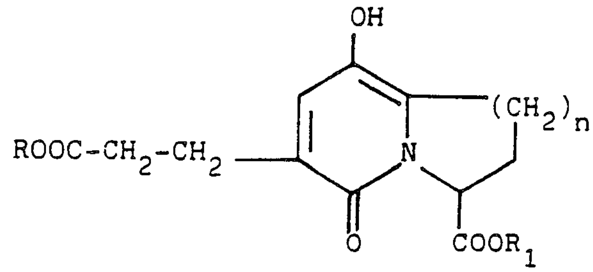

(I)

in which n is 1 or 2, R and $R_1$ may be the same or different, and are each hydrogen, $C_1$—$C_6$ alkyl, indan-5-yl, phthalidyl, or an acyloxymethyl group of the formula

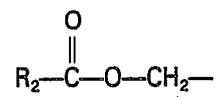

in which $R_2$ is $C_1$—$C_4$ alkyl, phenyl, halophenyl, or methylphenyl; or a pharmaceutically-acceptable salt thereof, which comprises culturing *Streptomyces chromofuscus* NRRL 15098 in a nutrient culture medium containing assimilable sources of carbon, nitrogen and inorganic salts under submerged aerobic fermentation conditions.

2. A process as claimed in claim 1 for preparing a compound of Formula (II) in which R and $R_1$ are defined as in Claim 1;

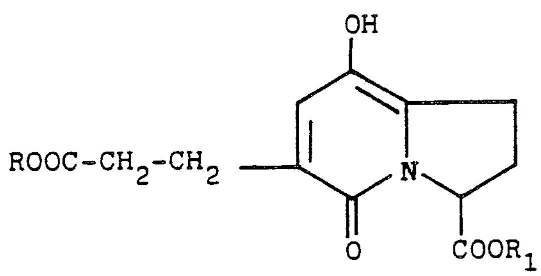

(II)

or a pharmaceutically-acceptable salt thereof.

3. A process as claimed in claim 1 for preparing A—58365 Factor A of Formula (III):

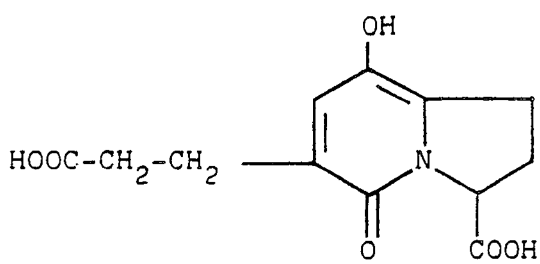

(III)

or a pharmaceutically-acceptable salt thereof.

4. A process as claimed in claim 1 for preparing a compound of Formula (IV) in which R and $R_1$ are defined as in claim 1;

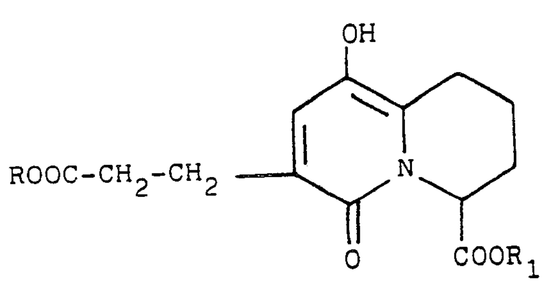

(IV)

or a pharmaceutically-acceptable salt thereof.

5. A process as claimed in claim 1 for preparing A—58365 Factor B of Formula (V):

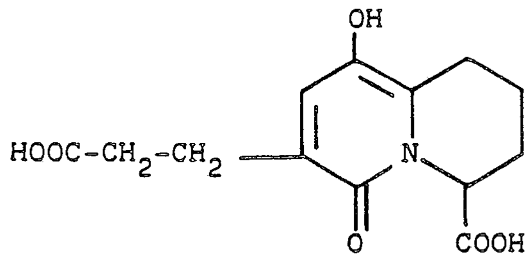

(V)

or a pharmaceutically-acceptable salt thereof.

6. A process as claimed in claim 1 for preparing A—58365 Factor C which

(a) has a molecular weight of 295;

(b) shows m/e 295 [$M^+$] in its field desorption mass spectrum;

(c) has an empirical formula of $C_{14}H_{17}NO_6$;

(d) a retention time of about 18.2 minutes upon reverse phase high performance liquid chromatography using a mobile phase of 6% acetonitrile, .3% formic acid and 93.7% water;

or a pharmaceutically-acceptable salt thereof.

7. A process for producing A—58365 factors A, B, or C which comprises culturing *Streptomyces chromofuscus* NRRL 15098 in a nutrient culture medium containing assimilable sources of carbon, nitrogen and inorganic salts under submerged aerobic fermentation conditions.

8. A process for producing A—58365 Factor A as claimed in claim 7 which comprises supplementing the culture medium with between about 1 to 6 g of proline per liter of medium.

9. A process for A—58365 Factor B as claimed in claim 7 which comprises supplementing the culture medium with between about 1 to 6 g of proline and 1 to 3 g of lysine per liter of medium.

10. A process as claimed in any one of claims 7 to 9 followed by isolating Factor A, Factor B or Factor C, and, optionally if desired, esterifying and/or salifying the product.

11. A biologically pure culture of *Streptomyces chromofuscus* NRRL 15098.

12. *Streptomyces chromofuscus* NRRL 15098.

**Patentansprüche für die Vertragsstaaten: BE CH DE FR GB IT LI LU NL SE**

1. Verbindung der Formel (I)

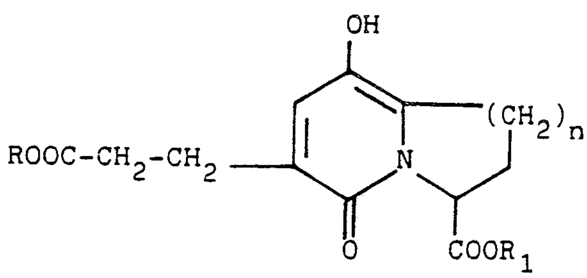

(I)

worin n für 1 oder 2 steht, R und $R_1$ gleich oder verschieden sein können und jeweils Wasserstoff, $C_1$—$C_6$-Alkyl, Indan-5-yl, Phthalidyl oder eine Acyloxymethylgruppe der Formel

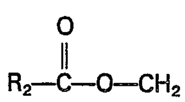

bedeuten, worin $R_2$ für $C_1$—$C_4$-Alkyl, Phenyl, Halogenphenyl oder Methylphenyl steht, oder ein pharmazeutisch annehmbares Salz hiervon.

2. Verbindung der Formel (II), worin R und $R_1$ wie im Anspruch 1 definiert sind,

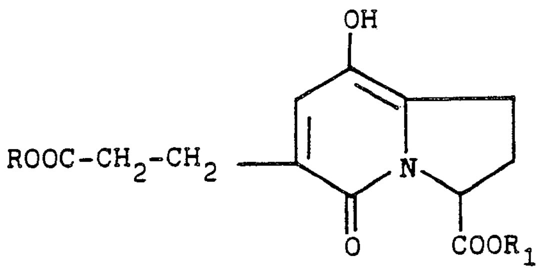

(II)

oder ein pharmazeutisch annehmbares Salz hiervon.

3. Faktor A von A—58365 der Formel (III)

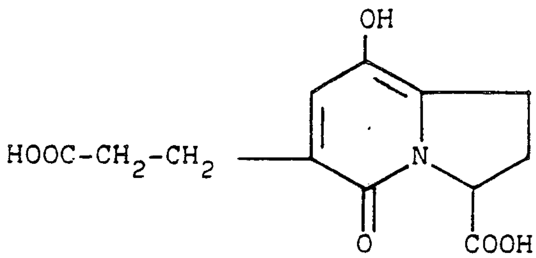

(III)

oder ein pharmazeutisch annehmbares Salz hiervon.

4. Verbindung der Formel (IV), worin R und $R_1$ wie im Anspruch 1 Definiert sind,

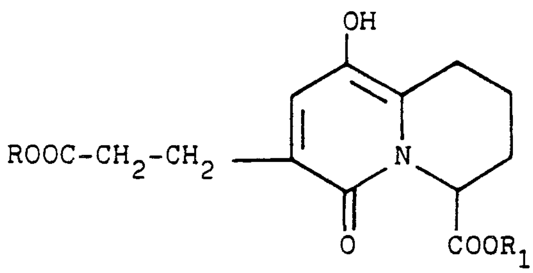

(IV)

oder ein pharmazeutisch annehmbares Salz hiervon.

5. Faktor B von A—58365 der Formel (V)

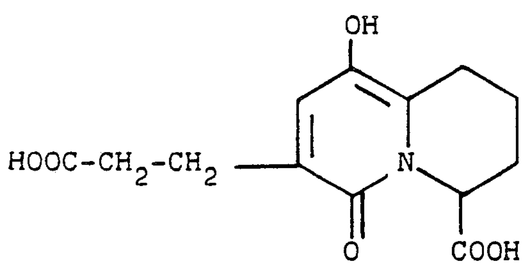

(V)

oder ein pharmazeutisch annehmbares Salz hiervon.

6. Faktor C von A—58365 mit

(a) einem Molekulargewicht von 295,

(b) einem m/e-Wert von 295 ($M^+$) im Felddesorptionsmassenspektrum,

(c) der empirischen Formel $C_{14}H_{17}NO_6$ und

(d) einer Retentionszeit von etwa 18,2 Minuten nach einer Hochleistungsflüssigkeitschromatographie mit Umkehrphase unter Anwendung einer mobilen Phase aus 6 Prozent Acetonitril, 0,3 Prozent Ameisensäure und 93,7 Prozent Wasser,

oder ein pharmazeutisch annehmbares Salz hiervon.

7. Verfahren zur Herstellung der Faktoren A, B oder C von A—58365, dadurch gekennzeichnet, daß Streptomyces chromofuscus NRRL 15098 in einem Nährkulturmedium, das assimilierbare Quellen für

Kohlenstoff, Stickstoff und anorganische Salze enthält, unter submersen aeroben Fermentationsbedingungen gezüchtet wird.

8. Verfahren zur Herstellung des Faktors A von A—58365 nach Anspruch 7, dadurch gekennzeichnet, daß das Kulturmedium mit etwa 1 bis 6 g Prolin pro Liter Medium ergänzt wird.

9. Verfahren zur Herstellung des Faktors B von A—58365 nach Anspruch 7, dadurch gekennzeichnet, daß das Kulturmedium mit etwa 1 bis 6 g Prolin und mit etwa 1 bis 3 g Lysin pro Liter Medium ergänzt wird.

10. Verfahren nach einem der Ansprüche 7 bis 9, dadurch gekennzeichnet, daß die Faktoren A, B oder C isoliert werden und das Produkt dann gewünschtenfalls einer Veresterung und/oder Salzbildung unterzogen wird.

11. Verbindung der Formel (I) nach einem der Ansprüche 1 bis 6 oder ein pharmazeutisch annehmbares Salz hiervon zur Verwendung als hypotensives Mittel.

12. Pharmazeutische Formulierung, dadurch gekennzeichnet, daß sie eine Verbindung der Formel (I) nach einem der Ansprüche 1 bis 6 oder ein pharmazeutisch annehmbares Salz hiervon als Wirkstoff in Verbindung mit einem oder mehreren pharmazeutisch annehmbaren Trägern oder Hilfsmitteln hierfür enthält.

13. Biologisch reine Kultur von Streptomyces chromofuscus NRRL 15098.

14. Streptomyces chromofuscus NRRL 15098.

**Patentansprüche für den Vertragsstaat: AT**

1. Verfahren zur Herstellung einer Verbindung der Formel (I)

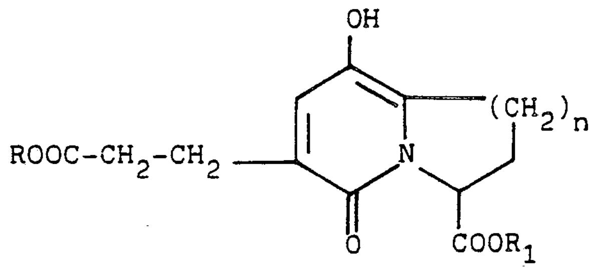

(I)

worin n für 1 oder 2 steht, R und $R_1$ gleich oder verschieden sein können und jeweils Wasserstoff, $C_1$—$C_6$-Alkyl, Indan-5-yl, Phthalidyl oder eine Acyloxymethylgruppe der Formel

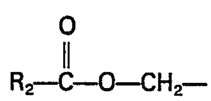

bedeuten, worin $R_2$ für $C_1$—$C_4$-Alkyl, Phenyl, Halogenphenyl oder Methylphenyl steht, oder eines pharmazeutisch annehmbaren Salzes hiervon, dadurch gekennzeichnet, daß Streptomyces chromofuscus NRRL 15098 in einem Nährkulturmedium, das assimilierbare Quellen für Kohlenstoff, Stickstoff und anorganische Salze enthält, unter submersen aeroben Fermentationsbedingungen gezüchtet wird.

2. Verfahren nach Anspruch 1 zur Herstellung einer Verbindung der Formel (II), worin R und $R_1$ wie im Anspruch 1 definiert sind,

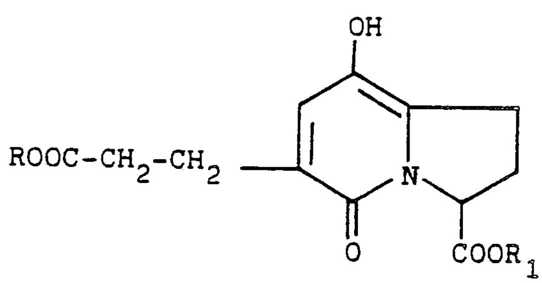

(II)

oder eines pharmazeutisch annehmbares Salzes hiervon.

3. Verfahren nach Anspruch 1 zur Herstellung des Faktors A von A—58365 der Formel (III)

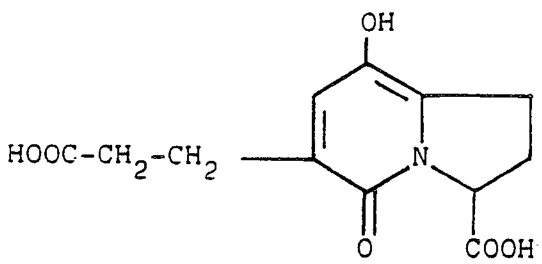

(III)

oder eines pharmazeutisch annehmbares Salzes hiervon.

4. Verfahren nach Anspruch 1 zur Herstellung einer Verbindung der Formel (IV), worin R und $R_1$ wie im Anspruch 1 definiert sind,

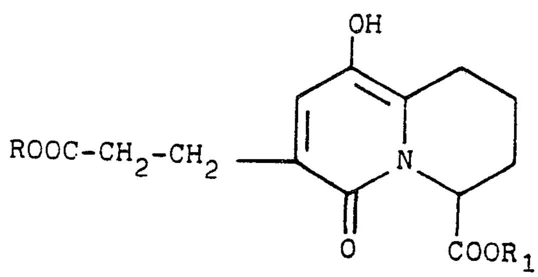

(IV)

oder eines pharmazeutisch annehmbares Salzes hiervon.

5. Verfahren nach Anspruch 1 zur Herstellung des Faktors B von A—58365 der Formel (V)

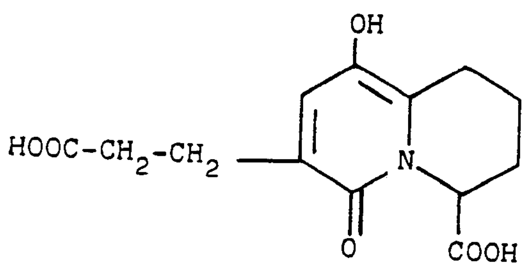

(V)

oder eines pharmazeutisch annehmbaren Salzes hiervon.

6. Verfahren nach Anspruch 1 zur Herstellung des Faktors C von A—58365 mit

(a) einem Molekulargewicht von 295,

(b) einem m/e-Wert von 295 ($M^+$) im Felddesorptionsmassenspektrum,

(c) der empirischen Formel $C_{14}H_{17}NO_6$ und

(d) einer Retentionszeit von etwa 18,2 Minuten nach einer Hochleistungsflüssigkeitschromatographie mit Umkehrphase unter Anwendung einer mobilen Phase aus 6 Prozent Acetonitril, 0,3 Prozent Ameisensäure und 93,7 Prozent Wasser,

oder eines pharmazeutisch annehmbaren Salzes hiervon.

7. Verfahren zur Herstellung der Faktoren A, B oder C von A—58365, dadurch gekennzeichnet, daß Streptomyces chromofuscus NRRL 15098 in einem Nährkulturmedium, das assimilierbare Quellen für Kohlenstoff, Stickstoff und anorganische Salze enthält, unter submersen aeroben Fermentationsbedingungen gezüchtet wird.

8. Verfahren zur Herstellung des Faktors A von A—58365 nach Anspruch 7, dadurch gekennzeichnet, daß das Kulturmedium mit etwa 1 bis 6 g Prolin pro Liter Medium ergänzt wird.

9. Verfahren zur Herstellung des Faktors B von A—58365 nach Anspruch 7, dadurch gekennzeichnet, daß das Kulturmedium mit etwa 1 bis 6 g Prolin und mit etwa 1 bis 3 g Lysin pro Liter Medium ergänzt wird.

10. Verfahren nach einem der Ansprüche 7 bis 9, dadurch gekennzeichnet, daß die Faktoren A, B oder C isoliert werden und das Produkt dann gewünschtenfalls einer Veresterung und/oder Salzbildung unterzogen wird.

11. Biologisch reine Kultur von Streptomyces chromofuscus NRRL 15098.

12. Streptomyces chromofuscus NRRL 15098.

**Revendications pour les Etats contractants: BE CH DE FR GB IT LI LU NL SE**

1. Composé de formule (I):

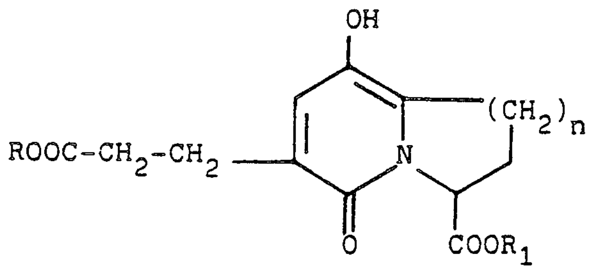

(I)

dans laquelle n représente 1 ou 2, R et $R_1$ peuvent être identiques ou différents et représentent chacun un atome d'hydrogène, un groupe alkyle en $C_1$—$C_6$, un groupe indan-5-yle, un groupe phtalidyle ou un groupe acyloxyméthyle de formule:

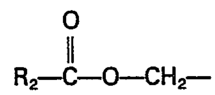

dans laquelle $R_2$ représente un groupe alkyle en $C_1$—$C_4$, un groupe phényle, un groupe halophényle, ou un groupe méthylphényle; ou encore un sel pharmaceutiquement acceptable de ce composé.

2. Composé de formule (II) dans laquelle R et $R_1$ ont les significations définies dans la revendication 1

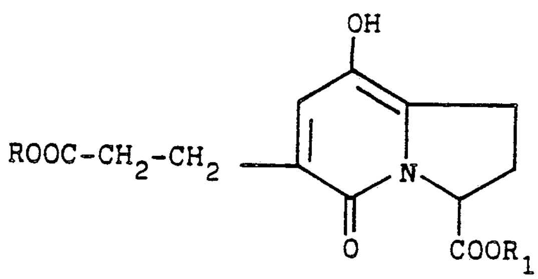

(II)

ou un sel pharmaceutiquement acceptable de ce composé.

3. Facteur A d'A—58365 de formule (III):

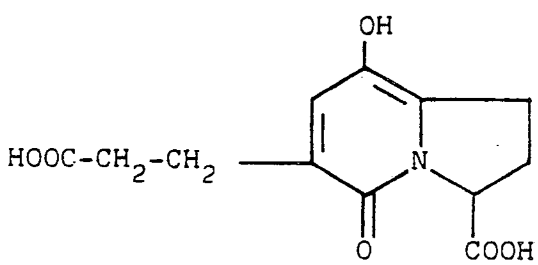

(III)

ou un de ses sels pharmaceutiquement acceptables.

4. Composé de formule (IV) dans laquelle R et $R_1$ ont les significations définies dans la revendication 1;

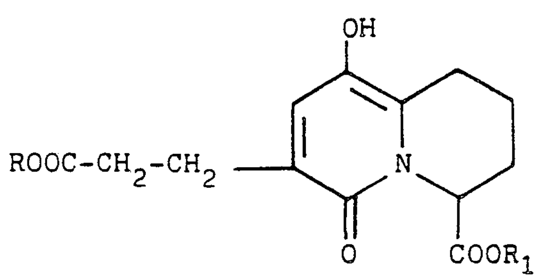

(IV)

ou un de ses sels pharmaceutiquement acceptables.

5. Facteur B d'A—58365 de formule (V):

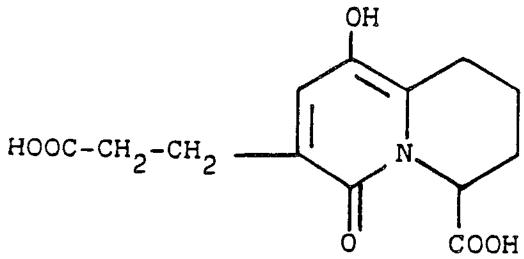

(V)

ou un de ses sels pharmaceutiquement acceptables.

6. Facteur C d'A—58365:

(a) qui a un poids moléculaire de 295;

(b) qui présente m/e 295 ($M^+$) dans son spectre de masse de désorption de champ;

(c) qui répond à la formule empirique $C_{14}H_{17}NO_6$;

(d) qui a un temps de rétention d'environ 18,2 minutes lors de la chromatographie liquide à haut rendement à phases inversées en utilisant une phase mobile de 6% d'acétonitrile, de 0,3% d'acide formique et de 93,7% d'eau;

ou un sel pharmaceutiquement acceptable de ce facteur.

7. Procédé de production des facteurs A, B ou C d'A—58365, caractérisé en ce qu'il consiste à cultiver Streptomyces chromofuscus NRRL 15098 dans un milieu de culture nutritif contenant des sources assimilables de carbone, d'azote et de sels inorganiques dans des conditions de fermentation aérobie submergée.

8. Procédé de préparation du facteur A d'A—58365 selon la revendication 7, caractérisé en ce qu'il consiste à compléter le milieu de culture avec environ 1 à 6 g de proline par litre de milieu.

9. Procédé de préparation du facteur B d'A—58365 selon la revendication 7, caractérisé en ce qu'il consiste à compléter le milieu de culture avec entre environ 1 et 6 g de proline et 1 à 3 g de lysine par litre de milieu.

10. Procédé selon l'une quelconque des revendications 7 à 9 suivi de l'isolation du facteur A, du facteur B ou du facteur C et éventuellement, si ou le désire, de l'estérification et/ou de la salification du produit.

11. Composé de formule (I) selon l'une quelconque des revendications 1 à 6, ou un de ses sels pharmaceutiquement acceptables, en vue de les utiliser comme agents hypotenseurs.

12. Formulation pharmaceutique, caractérisée en ce qu'elle comprend, comme ingrédient actif, un composé de formule (I) selon l'une quelconque des revendications 1 à 6, ou un de ses sels pharmaceutiquement acceptables, en association avec un ou plusieurs véhicules ou supports pharmaceutiquement acceptables pour ce composé ou ce sel.

13. Culture biologiquement pure de Streptomyces chromofuscus NRRL 15098.

14. Streptomyces chromofuscus NRRL 15098.

**Revendications pour l'Etat contractant: AT**

1. Procédé de préparation d'un composé de formule (I):

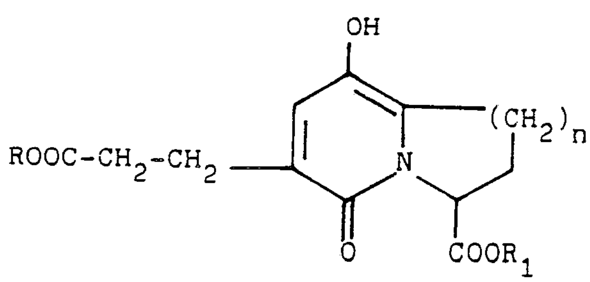

(I)

dans laquelle n représente 1 ou 2, R et $R_1$ peuvent être identiques ou différents et représentent chacun un atome d'hydrogène, un groupe alkyle en $C_1$—$C_6$, un groupe indan-5-yle, un groupe phtalidyle ou un groupe acyloxyméthyle de formule:

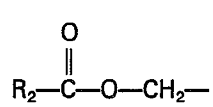

dans laquelle $R_2$ représente un groupe alkyle en $C_1$—$C_4$, un groupe phényle, un groupe halophényle ou un groupe méthylphényle; ou d'un de ses sels pharmaceutiquement acceptables, caractérisé en ce qu'il consiste à cultiver Streptomyces chromofuscus NRRL 15098 dans un milieu de culture nutritif contenant des sources assimilables de carbone, d'azote et de sels inorganiques dans des conditions de fermentation aérobie submergée.

2. Procédé selon la revendication 1 pour la préparation d'un composé de formule (II) dans laquelle R et $R_1$ ont les significations définies dans la revendication 1

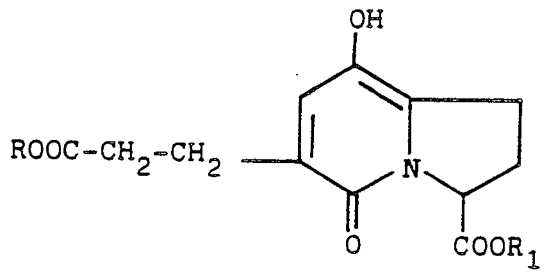

(II)

ou d'un de ses sels pharmaceutiquement acceptables.

3. Procédé selon la revendication 1 pour la préparation du facteur A d'A—59365 de formule (III):

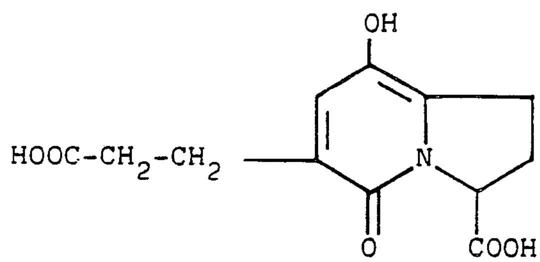

(III)

ou d'un de ses sels pharmaceutiquement acceptables.

4. Procédé selon la revendication 1 pour la préparation d'un composé de formule (IV) dans laquelle R et $R_1$ ont les significations définies dans la revendication 1

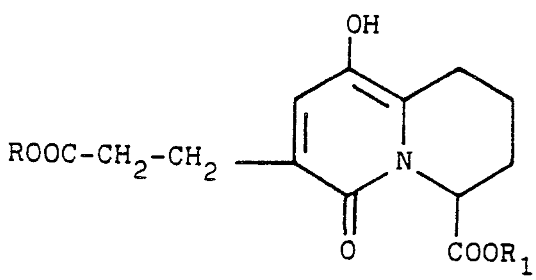

(IV)

ou d'un de ses sels pharmaceutiquement acceptables.

5. Procédé selon la revendication 1 pour la préparation du facteur B d'A—58365 de formule (V):

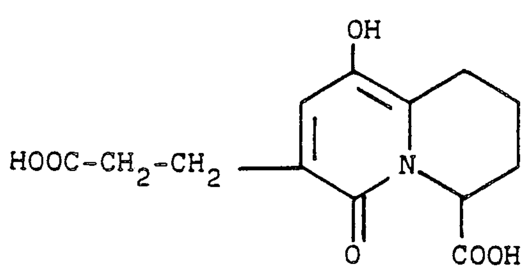

(V)

ou d'un de ses sels pharmaceutiquement acceptables.

6. Procédé selon la revendication 1 pour la préparation du facteur C d'A—58365:

(a) qui a un poids moléculaire de 295;

(b) qui présente m/e 295 ($M^+$) dans son spectre de masse de désorption de champ;

(c) qui répond à la formule empirique $C_{14}H_{17}NO_6$;

(d) qui a un temps de rétention d'environ 18,2 minutes lors de la chromatographie liquide à haut rendement à phases inversées en utilisant une phase mobile de 6% d'acétonitrile, de 0,3% d'acide formique et de 93,7% d'eau;

ou d'un de ses sels pharmaceutiquement acceptables.

7. Procédé pour la production des facteurs A, B ou C d'A—58365, caractérisé en ce qu'il consiste à cultiver Streptomyces chromofuscus NRRL 15098 dans un milieu de culture nutritif contenant des sources assimilables de carbone, d'azote et de sels inorganiques dans des conditions de fermentation aérobie submergée.

8. Procédé de préparation du facteur A d'A—58365 selon la revendication 7, caractérisé en ce qu'il consiste à compléter le milieu de culture avec entre environ 1 et 6 g de proline par litre de milieu.

9. Procédé de préparation du facteur B d'A—58365 selon la revendication 7, caractérisé en ce qu'il consiste à compléter le milieu de culture avec entre environ 1 et 6 g de proline et 1 à 3 g de lysine par litre de milieu.

10. Procédé selon l'une quelconque des revendications 7 à 9 suivi de l'isolation du facteur A, du facteur B ou du facteur C et éventuellement, si ou le désire, de l'estérification et/ou de la salification du produit.

11. Culture biologiquement pure de Streptomyces chromofuscus NRRL 15098.

12. Streptomyces chromofuscus NRRL 15098.